Europäisches Patentamt

European Patent Office

Office européen des brevets

(19)

(11) Publication number: 0 387 697
A2

# EUROPEAN PATENT APPLICATION

(21) Application number: 90104414.9

(22) Date of filing: 08.03.90

(51) Int. Cl.5: **C12Q 1/52, C12Q 1/32, C12Q 1/26**

(30) Priority: 13.03.89 US 337249

(43) Date of publication of application:
**19.09.90 Bulletin 90/38**

(84) Designated Contracting States:
**DE ES FR IT**

(71) Applicant: **ABBOTT LABORATORIES**
**CHAD-0377, AP6D/2 One Abbott Park Road**
**Abbott Park, IL 60064-2204(US)**

(72) Inventor: **Yost, David A.**
**96 Cedar Drive**

**Round Lake Park, Illinois 60073(US)**
Inventor: **Dolan, Peggy Anne**
**134 Main St., Apt. 3**
**Evanston, Illinois 60202(US)**
Inventor: **Pennington, Charles D.**
**552 Pam Court**
**Wheeling, Illinois 60090(US)**

(74) Representative: **Modiano, Guido et al**
**MODIANO, JOSIF, PISANTY & STAUB**
**Modiano & Associati Via Meravigli, 16**
**I-20123 Milano(IT)**

(54) **Determination of aminotranferases.**

(57) A method and reagent system for determining the amount of an aminotransferase present in a biological test sample. The aminotransferase is determined by contacting a test sample containing an aminotransferase with its corresponding amino acid, a ketoacid, a dehydrogenase or a reductase, and a reduced form of thionicotinamide adenine dinculeotide or an analog thereof to form a liquid mixture thereof. The visible absorbance of the liquid mixture is measured and correlated to the amount of aminotransferase present in the test sample. The method and reagent system of the present invention are particularly useful for determining alanine aminotransferase and aspartate aminotransferase.

EP 0 387 697 A2

FIG. 1

## DETERMINATION OF AMINOTRANSFERASES

### Background of the Invention

The present invention relates to the determination of the amount of an aminotransferase present in a liquid test sample. In particular, the present invention relates to a method and reagent system for the spectrophotometric determination of an aminotransferase in a biological fluid for the diagnosis of various pathological conditions.

The determination of aminotransferases is particularly useful for diagnosing a number of pathological conditions associated with tissue damage. For example, the clinical significance of alanine aminotransferase (ALT) and aspartate aminotransferase (AST) have been associated with lever disease involving hepatic necrosis, such as viral hepatitis and alcoholic hepatitis, and increased levels of ALT have been used as a surrogate marker for hepatitis Non-A and hepatitis Non-B.

In addition, primary or metastatic carcinomas of the liver usually result in elevations of both ALT and AST serum levels of at least about five fold more than their normal serum levels. Although pathological conditions which affect the liver or liver cell integrity will result in an elevation of both ALT and AST serum levels, AST is particularly associated with pathological conditions involving damage to tissues of the heart, such as myocardial infarction.

The determination of aminotransferases in a biological fluid involves coupled oxidation-reduction reactions with a dehydrogenase or reductase which are catalyzed by ALT or AST wherein the transfer of amino groups between the respective amino acids, i.e., alanine or aspartate, respectively, and an oxoacid is monitored. For example, it has been shown that a variety of nicotinamide adenine dinucleotide analogs can be used as coenzymes in dehydrogenase reactions (see, for example, Anderson, B.M., et al., (1958) J. Biol. Chem., 234 1226-1232). The absorption maxima for the reduced form of thionicotinamide adenine dinucleotide at 400 nm for the analysis of lactate dehydrogenase has been described (Minato, S., et al., (1976) Clinica Chimica Acta, 69, 243-249). However, such analysis is based upon the slow reduction of oxidized thionicotinamide adenine dinucleotide to the reduced form thereof. The reactivity of thionicotinamide analogs of NAD and NADP in various dehydrogenase systems (Anderson, M., et al., (1963) Biochemistry, 2, 1017) have also been described.

Generally, oxoacids formed by the catalytic activity of aminotransaminases can be determined by measuring the change in absorbance which results from the catalytic reduction to their corresponding hydroxyacids through the oxidation of nicotinamide adenine dionucleotide (NADH) or nicotinamide adenine dinucleotide $2'$-diphosphate (NADPH), to $NAD^+$ or $NADP^+$, respectively, as follows:

(a) aspartate aminotransferase + aspartate + alpha-ketoglutarate $\rightleftharpoons$ oxaloacetate + glutamate
oxaloacetate + NADH + malate dehydrogenase $\rightleftharpoons$ malate + $NAD^+$

(b) alanine aminotransferase + alanine + alpha-ketoglutarate $\rightleftharpoons$ pyruvate + glutamate
pyruvate + NADH + lactate dehydrogenase $\rightleftharpoons$ lactate + $NAD^+$

However, such change in absorbance between NADH and $NAD^+$ must be measured at an ultraviolet (UV) wavelength of about 340 nm. Accordingly, such methods require expensive analytical instruments which are capable of measuring such changes in the U.V. absorbance spectra region and therefore are not adaptable to the less expensive analytical systems based on measurements in the visible absorption spectra region which are more readily available in the clinical laboratory.

### Summary of the Invention

The present invention provides a method and reagent system for determining the amount of an aminotransferase present in a liquid test sample employing nicotinamide adenine dinucleotide analogs which are capable of providing a change in absorbance between the reduced and oxidized forms thereof which can be measured in the visible spectra region. In particular, the method according to the present invention is carried out by contacting a liquid test sample containing the amino transferase to be determined, the corresponding amino acid, a ketoacid, a dehydrogenase or a reductase, and a nicotinamide adenine dinucleotide analog, preferably thionicotinamide adenine dinucleotide (SNADH), which is capable of providing a measurable change of absorption in the visible absorption spectra upon the oxidation thereof. The visible absorbance of the liquid mixture is measured at from between about 360 nm and about 450 nm, preferably at from between about 390 nm and about 415 nm, more preferably at about 400 nm.

For example, in the case of determining the amount of aspartate aminotransferase present in a liquid test sample, the liquid test sample, which is usually in the form of a biological fluid or diluent thereof, such as whole blood, serum, plasma, urine, saliva, and the like, is preferably contacted with alpha-ketoglutarate and aspartate to form oxaloacetate and glutamate by the catalytic activity of aspartate aminotransferase, and then the formed oxaloacetate reacts with SNADH and reduced to malate by the catalytic acivity of malate dehydrogenase. Similarly, in the case of determining the amount of alanine aminotransferase present in a liquid test sample, the liquid test sample is preferably contacted with alpha-ketoglutarate and alanine to form pyruvate and glutamate by the catalytic activity of alanine aminotransferase, and then the formed pyruvate is reacted with SNADH and reduced to lactate by the catalytic activity of lactate dehydrogenase. In either case, the SNADH and the coupling enzymes are present in sufficient amounts so that only the alanine aminotransferase and the aspartate aminotransferase to be measured are rate limiting wherein as the catalytic couple continues, SNADH is oxidized to $SNAD^+$ as a function of the amount of the respective aminotransferase present in the liquid test sample and the visible absorbance thereof measured as described above.

Accordingly, since the oxidation of SNADH provides a measurable change in absorbance which can be measured in the visible absorbance spectra region, the method of the present invention is particularly adaptable to visible absorbance systems commonly found ih the clinical laboratory. Moreover, since the oxidized form of SNADH provides about a two-fold increase in signal over the amount of signal provided by the oxidized form of NADH, about one-half the amount of SNADH is required to perform the method according to the present invention.

## Brief Description of the Drawings

Fig. 1 is a graph which illustrates the comparison of the rate of absorbance at various wavelengths in an assay for alanine aminotransferase according to the method of the present invention.

Fig. 2 is a graph which illustrates the rate of absorbance in an assay for alanine aminotransferase performed on an automated instrument according to the method of the present invention.

Fig. 3 is a graph which illustrates the serum correlation in an assay for alanine aminotransferase according to the method of the present invention.

## Description of the Preferred Embodiments

In carrying out the method of the present invention, an assay reagent for an aminotransferase to be determined is prepared comprising a reduced form of a nicotinamide adenine dinucleotide (NADH) analog as described above, the corresponding amino acid for the aminotransferase under determination, a ketoacid, and a dehydrogenase or a reductase, in a buffer system. In addition, the aminotransferase assay reagent further comprises a coenzyme, such as pyridoxyl phosphate, and the like, which is required for such transaminase reactions. The aminotransferase assay reagent is contacted with the test sample to form a liquid mixture wherein the oxidation of the NADH analog is oxidized as a function of the amount of the aminotransferase present in the test sample. The visible absorbance of the liquid mixture is then correlated to the amount of the aminotransferase present in the test sample according to methods known in the art.

Preferably, the NADH analog is a reduced form of thionicotinamide adenine dinucleotide (SNADH). It is to be understood, that since the determination of aminotransferase according to the present invention is based upon absorption measurements in the visible spectra region, other analogs of NADH which possess such spectral properties can be selected by one skilled in the art apprised of the foregoing consideration and as set forth below.

The amino acid component in the aminotransferase assay reagent will of course depend upon the particular aminotransferase under determination. Accordingly, the method according to the present invention can be followed for the determination of a variety of aminotransferases wherein the corresponding amino acid can be selected from the group consisting of alanine, aspartate, tyrosine, asparagine, glutamine, cystine, and the like.

As will be understood by one skilled in the art, the ketoacid component and the dehydrogenase or reductase component will also depend upon the particular aminotransferase under determination. Accordingly, depending upon the aminotransferase, the ketoacid can be selected from a number of ketoacids known in the art and include, but are not intended to be limited to, alpha-ketoglutarate, such as for the determination of alanine or aspartate; alpha-ketosuccinamic acid, such as for the determination of asparagine; alpha-ketoglutaramic acid for the determination of glutamine; and the like. Similarly, the dehydrogenase or reductase component can be selected from a number of dehydrogenases and reductases known in the art and include, but are not intended to be limited to, lactate dehydrogenase, malate dehydrogenase, dihydropteridine reductase, and the like.

It is to be understood that although the method according to the present invention is preferably carried out by simultaneously contacting a test sample with all of the components of the aminotransferase assay reagent as described above, the method according to the present invention can also be carried out by sequentially contacting a test sample with the components of the aminotransferase assay reagent in a desired ordered sequence and/or combination. For example, the test sample can first be sequentially contacted with (i) the amino acid corresponding to the aminotransferase under determination and the ketoacid to form a first liquid mixture, and then (ii) the first liquid mixture can be contacted with SNADH and the dehydrogenase or reductase to form a second liquid mixture wherein the visible absorbance thereof is measured and correlated to the amount of the aminotransferase present in the test sample.

As described above, the method of the present invention is particularly useful for determining alanine aminotransferase and aspartate aminotransferase in a liquid test sample. For the determination of alanine aminotransferase (ALT), the ALT assay reagent preferably comprises SNADH, L-alanine, alpha-ketoglutarate, pyridoxyl phosphate, a buffer system comprising tris(hydroxymethyl)aminoethane and succinic acid, and lactate dehydrogenase, preferably derived from beef heart. Similarly, for the determination of aspartate aminotransferase (AST), the AST assay reagent preferably comprises SNADH, L-aspartate, alpha-ketoglutarate, pyridoxyl phosphate, a buffer system comprising tris(hydroxymethyl)aminoethane and succinic acid, and malate dehydrogenase. Preferably, the AST assay reagent also comprises lactate dehydrogenase which reacts with residual pyruvate which may be present and which would otherwise interfere with the desired reactions for the determination of AST.

The aminotransferase assay reagents prepared according to the present invention can be in the form of a liquid reagent or reagents, or can be in the form of a powder, lyophilized, or otherwise presented in a dried form thereof. The preferred relative concentrations for the ALT assay reagent and the AST assay reagent are set forth in Table 1. It is to be understood, however, that such relative concentrations are not intended to be limited to those set forth in Table 1 and, accordingly, variations thereof can be determined by one skilled in the art apprised of the foregoing considerations without departing from the teachings of the present invention.

Table 1

|  | ALT | AST |
|---|---|---|
|  | (92 mg reconstituted in 1 mL $H_2O$) | (83 mg reconstituted in 1 mL $H_2O$) |
| SNADH | 0.12 mM/L | 0.12 mM/L |
| L-Alanine | 500 mM/L | 0.0 |
| L-Aspartate | 0.0 | 309 mM/L |
| Alpha-Ketoglutarate | 15.0 mM/L | 12.0 mM/L |
| Pyridoxyl Phosphate | 0.10 mM/L | 0.10 mM/L |
| Tris(hydroxymethyl)aminoethane | 100 mM/L | 80 mM/L |
| Succinic Acid | 30.3 mM/L | 20.3 mM/L |
| Lactate Dehydrogenase | 12,000 U/L | 9,000 U/L |
| Malate Dehydrogenase | 0.0 | 420 U/L |

The method according to the present invention and assay reagents employed therewith as described above is particularly useful and readily adaptable to a variety of automated diagnostic instruments known in the art which are capable of measuring changes in abosrbance in the visible spectral region. For example, an automated aminotransferase assay according to the method of the present invention can be carried out employing an Abbott VP™ Bichromatic Analyzer (Abbott Laboratories, Abbott Park, Illinois, USA) which can be programmed into the user area of the software therefore by one skilled in the art. Adaptation of the assay according to the present invention for the determination of, for example, ALT, can be achieved by programming the following parameters: a 415/550 nm filter; a 1:11 sample dilution; a 6 minute incubation time; and an incubation temperature of 37°C. The results are provided in IU/L, and ALT activities can be measured from 0.0 to 200 IU/L. Similarly, an automated aminotransferase assay according to the method of the present invention can be carried out employing an Abbott Commander® Parallel Processing Center (PPC, Abbott Laboratories, Abbott Park, IL, USA), such as for the determination of ALT as specifically set forth in Example 4 below.

The reagent system of the present invention comprises all of the essential elements or assay reagents as described above required to conduct a desired aminotransferase assay encompassed by the present invention. The reagent system is presented in a commercially packaged form, as a composition or admixture where the compatability of the reagents will allow, in a test device configuration, or as a test kit, i.e., a packaged combination of one or more containers holding the necessary reagents. Included in the reagent system are the reagents appropriate for the desired aminotransferase assay system, and can include other materials as are known in the art and which may be desirable from a commercial and user standpoint, such as buffers, diluents, standards, and the like. Particularly preferred is a reagent system for the determination of ALT or AST according to the present invention.

The present invention will now be illustrated, but is not intended to be limited, by the following examples.

## Example 1

### Preparation of Thionicotinamide Adenine Dinucleotide

Thionicotinamide adenine dinucleotide ($SNAD^+$) was synthesized enzymatically by utilizing nicotinamide adenine dinucleotide-ase (NADase). The enzymatic reaction catalyzed by NADase involves a base exchange reaction where the pyridine moiety of $NAD^+$ can be replaced with a pyridine analog or another nucleophilic group. The source of the NADase was from pig brain (acetone powder, Sigma Chemical Co., USA, Catalog No. B-6503) or from snake venom (Sigma Catalog No. V-6500). Snake venom NADase was used in either the crude form or purified as described by Yost, et. al., [J. Biol. Chem., 256, 3647 (1981)].

$SNAD^+$ was purified on an anion exchange resin column (Dowex AG, 1-X8) and the sample applied to the column and followed by washing the column with water to remove residual thionicotinamide. The $SNAD^+$ was eluted using lithium formate and fractions which contained $SNAD^+$ were pooled and lyophilized. The lyophilized sample was dissolved in a small amount of water and was desalted on a Sephadex G-10 column. Fractions which contained $SNAD^+$ were pooled and lyophilized. The purified $SNAD^+$ was reduced either enzymatically, by the use of a dehydrogenase, or chemically by the use of a reducing agent, such as sodium dithionite. The SNADH was purified by ion exchange chromatography.

$NAD^+$ (3.0 mM), thionicotinamide (100 mM), and NADase (0.1 u/mL) were incubated in a buffered solution. The reaction was followed by removing an aliquot of the reaction mixture and the subsequent reduction by using horse liver alcohol dehydrogenase. The reduced product, SNADH, has an absorbance maximum at 400 nm while NADH has an absorbance maximum at 340 nm. The rate of product formation was therefore determined by following the increase in absorbance at 400 nm of the reduced species. The reaction was stopped when the absorbance at 400 nm reached its maximum value.

## Example 2

### Preparation of Alanine Aminotransferase (ALT) Assay Reagent

An assay reagent for determining ALT according to the present invention employing SNADH prepared according to Example 1 was prepared in the form of a powder which, when 92 mg thereof was reconstituted with 1 mL of water, resulted in the following concentrations of the active ingredients (pH 8.0):

| SNADH | 0.12 mM/L |
|---|---|
| L-Alanine | 500 mM/L |
| Alpha-ketoglutarate | 15 mM/L |
| Pyridoxyl phosphate | 0.10 mM/L |
| Tris(hydroxymethyl)aminoethane | 100 mM/L |
| Succinic acid | 30.3 mM/L |
| Rabbit Muscle Lactate Dehydrogenase | 12,000 U/L |

The ALT assay reagent further included, as inactive ingredients, fillers and enzyme stabilizers which did not affect the ALT activity. Alternatively, the ALT reagent can also be prepared using beef heart lactate dehydrogenase (LDH), having a final assay concentration of 9,000 U/L; or D-LDH (microbial source) having a final concentration of 9,000 U/L.

Example 3

ALT Assay

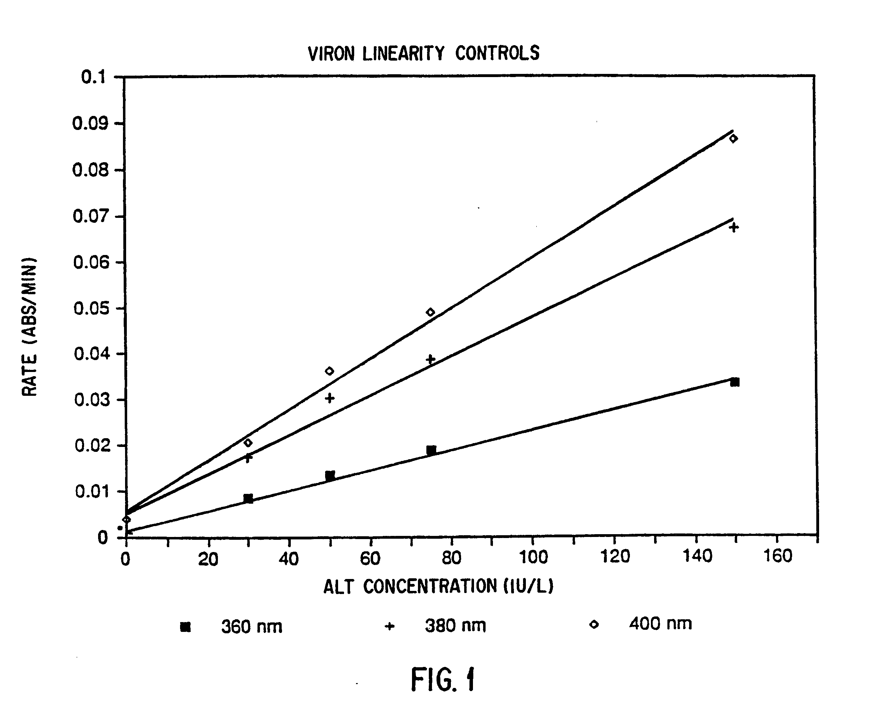

An assay for ALT employing the ALT reagent prepared according to Example 2 was performed on a spectrophotometer and monitored at various wavelengths. The ALT reagent was prepared utilizing beef heart LDH. 100 Microliters of a Viron ALT enzyme linearity set levels I-IV (Viron Catalog No. V-A2000) were pipetted into cuvettes containing 1 milliliter of the ALT reagent. The assay mixture was incubated for 3 minutes at 37° C and the absorbances were measured at 360 nm, 380 nm, and 400 nm at varying time intervals for 2 minutes. Rate calculations were compared to the known ALT concentration values (Fig. 1).

Example 4

Automated ALT Assay

An automated ALT assay was performed employing an Abbott Commander Parallel Processing Center (PPC, Abbott Laboratories, Abbott Park, IL, USA), an automated instrument for the processing of enzyme immunoassays. The PPC has multiple visible wavelength capabilities that can measure differential absorbances from sample wells. The PPC is designed to perform automated, precision reagent dispensing, bead washing, in-the-well spectrophotometric readings and data reduction for specific Abbott Laboratories assays.

In performing an assay on the PPC, a reagent is added directly to samples in a reaction tray and allowed to incubate before advancing to a read station. A series of bichromatic readings of each sample are taken to determine the rate of change in absorbance. A serum calibrator of known ALT concentration is analyzed simultaneously and employed in calculations to determine the ALT concentration of each sample.

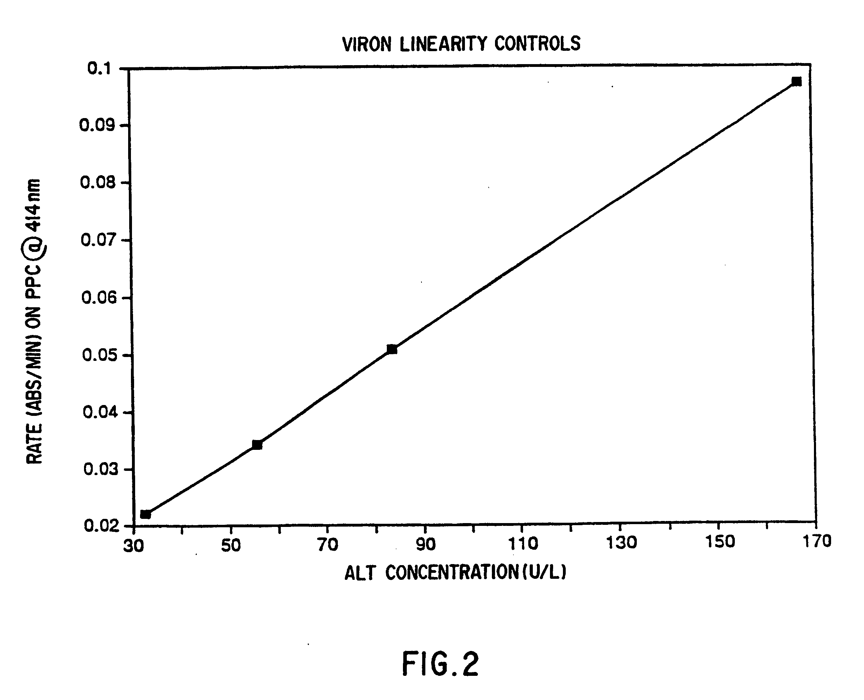

60 microliters of Viron ALT enzyme controls (Viron Catalog No. V-A2000) were pipetted into wells of a reaction tray followed by 600 microliters of the ALT reagent prepared according to Example 2 and automatically pipetted by the PPC. The assay mixtures were incubated for 3 minutes and a series of absorbance measurements at 415/600 nm were taken over a period of 1 minute. Rates were determined and compared to that of the known ALT concentration values (Fig. 2).

Example 5

## Comparison of ALT Assay Reagents

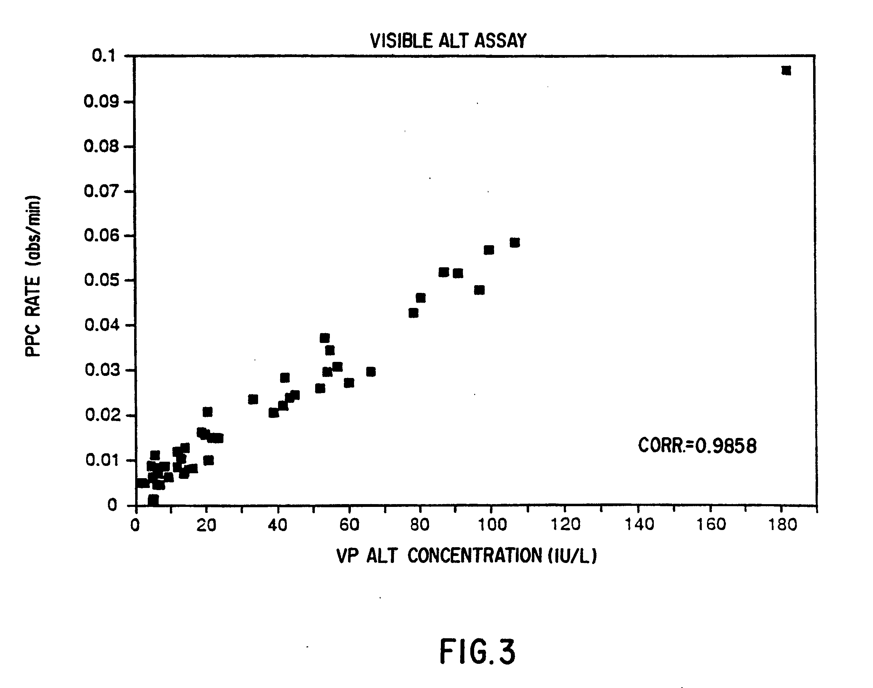

50 serum samples were assayed on an Abbott VP system using Abbott A-gent® ALT (Abbott Laboratories, Abbott Park, IL, USA; Catalog No. 6016) and the specified assay procedures. These values were compared to those obtained from the Commander PPC procedure as described above in Example 4 using the reagent prepared according to Example 2. A correlation coefficient of 0.9858 was obtained (Fig. 3).

It will be apparent that many modifications and variations of the invention as herein set forth are possible without departing from the spirit and scope thereof, and that, accordingly, such limitations are imposed only as indicated by the appended claims.

## Claims

1. A method for determining the amount of an aminotransferase present in a test sample, said method characterized by the steps of:
(a) contacting said test sample containing said aminotransferase with assay components comprising:
(i) an amino acid corresponding to said aminotransferase,
(ii) a ketoacid,
(iii) a dehydrogenase or a reductase, and
(iv) a reduced form of thionicotinamide adenine dinucleotide or an analog thereof,
wherein a liquid mixture is thereby formed, said reduced form of thionicotinamide adenine dinucleotide or analog thereof being oxidized as a function of the amount of said aminotransferase present is said liquid test sample; and
(b) measuring and correlating the visible absorbance of said liquid mixture to the amount of said aminotransferase present in said test sample.

2. The method of Claim 1 wherein said amino acid is selected from the group consisting of aspartate, alanine, tyrosine, asparagine, glutamine and cystine.

3. The method of Claim 1 wherein said ketoacid is selected from the group consisting of alpha-ketoglutarate, alpha-ketosuccinamic acid, and alpha-ketoglutaramic acid.

4. The method of Claim 1 wherein said amino acid is aspartate, said aminotransferase is aspartate aminotransferase, and said ketoacid is alpha-ketoglutarate.

5. The method of Claim 1 wherein said amino acid is alanine, said aminotransferase is alanine aminotransferase, and said ketoacid is alpha-ketoglutarate.

6. The method of any one of Claims 1-5 wherein said liquid mixture comprises a dehydrogenase selected from the group consisting of lactate dehydrogenase and malate dehydrogenase.

7. The method of any one of Claims 1-6 wherein said liquid mixture comprises a reductase.

8. The method of any one of Claims 1-7 wherein said visible absorbance is measured at or greater than about 360 nm.

9. The method of any one of Claims 1-8 wherein said visible absorbance is measured at from between about 390 nm and 450 nm.

10. A reagent system according to any one of Claims 1-9 for determining the amount of an aminotransferase in a test sample involving oxidation and reduction reactions which are measured in the visible absorption spectra region, said reagent system characterized by:
(a) an amino acid corresponding to said aminotransferase;
(b) a ketoacid;
(c) a dehydrogenase or a reductase; and
(d) a reduced form of thionicotinamide adenine dinucleotide or an analog thereof.

VIRON LINEARITY CONTROLS

FIG. 1

EP 0 387 697 A2

VIRON LINEARITY CONTROLS

FIG.2

EP 0 387 697 A2

VISIBLE ALT ASSAY

CORR.=0.9858

PPC RATE (obs/min)

VP ALT CONCENTRATION (IU/L)

FIG.3